# EUROPEAN PATENT SPECIFICATION

(11) **EP 2 249 869 B1**
(45) Date of publication and mention of the grant of the patent: **07.09.2011**
(21) Application number: 09708298.6
(22) Date of filing: 09.02.2009
(51) Int. Cl.: A61K 47/10, A61K 47/18, A61K 38/24, A61K 9/00

(54) **LIQUID FORMULATION OF FSH**
FLÜSSIGE FORMULIERUNG VON FSH
FORMULATION LIQUIDE DE FSH

(30) Priority: 08.02.2008 EP 08151231
(43) Date of publication of application: 17.11.2010
(73) Proprietor: BioGeneriX AG, 68199 Mannheim (DE)
(72) Inventor: STOLZENBERGER, Sascha, 64347 Griesheim (DE); KOHLER, Erich, 64646 Heppenheim (DE)
(74) Representative: Neuefeind, Regina
(86) International application number: PCT/EP2009/051451
(87) International publication number: WO 2009/098318

(56) References cited:
- WO-A-00/04913
- WO-A-2007/037607
- US-A- 5 503 827

### SEQUENCE LISTING

<110> Biogenerix AG
<120> liquid formulation of FSH
<130> B 8299
<160> 6
<170> PatentIn version 3.3
<210> 1
   <211> 116
   <212> PRT
   <213> Homo sapiens
<220>
   <221> CHAIN
   <222> (1)..(116)
   <223> alfa-chain of human FSH
<400> 1
   Met Asp Tyr Tyr Arg Lys Tyr Ala Ala Ile Phe Leu Val Thr Leu Ser
   1 5 10 15
   Val Phe Leu His Val Leu His Ser Ala Pro Asp Val Gln Asp Cys Pro
   20 25 30
   Glu Cys Thr Leu Gln Glu Asn Pro Phe Phe Ser Gln Pro Gly Ala Pro
   35 40 45
   Ile Leu Gln Cys Met Gly Cys Cys Phe Ser Arg Ala Tyr Pro Thr Pro
   50 55 60
   Leu Arg Ser Lys Lys Thr Met Leu Val Gln Lys Asn Val Thr Ser Glu
   65 70 75 80
   Ser Thr Cys Cys Val Ala Lys Ser Tyr Asn Arg Val Thr Val Met Gly
   85 90 95
   Gly Phe Lys Val Glu Asn His Thr Ala Cys His Cys Ser Thr Cys Tyr
   100 105 110
   Tyr His Lys Ser
   115
<210> 2
   <211> 129
   <212> PRT
   <213> Homo sapiens
<220>
   <221> CHAIN
   <222> (1)..(129)
   <223> beta-chain of human FSH
<400> 2
   Met Lys Thr Leu Gln Phe Phe Phe Leu Phe Cys Cys Trp Lys Ala Ile
   1               5                   10                  15
   Cys Cys Asn Ser Cys Glu Leu Thr Asn Ile Thr Ile Ala Ile Glu Lys
               20                  25                  30
   Glu Glu Cys Arg Phe Cys Ile Ser Ile Asn Thr Thr Trp Cys Ala Gly
           35                  40                  45
   Tyr Cys Tyr Thr Arg Asp Leu Val Tyr Lys Asp Pro Ala Arg Pro Lys
       50                  55                  60
   Ile Gln Lys Thr Cys Thr Phe Lys Glu Leu Val Tyr Glu Thr Val Arg
   65                  70                  75                  80
   Val Pro Gly Cys Ala His His Ala Asp Ser Leu Tyr Thr Tyr Pro Val
                   85                  90                  95
   Ala Thr Gln Cys His Cys Gly Lys Cys Asp Ser Asp Ser Thr Asp Cys
               100                 105                 110
   Thr Val Arg Gly Leu Gly Pro Ser Tyr Cys Ser Phe Gly Glu Met Lys
           115                 120                 125
   Glu
<210> 3
   <211> 369
   <212> DNA
   <213> Homo sapiens
<220>
   <221> misc_feature
   <223> wild-type nucleic acid sequence coding for the alfa-chain of human FSH
<400> 3
   cttaattaag ccgccagcat ggattactac agaaaatatg cagctatctt tctggtcaca      60
   ttgtcggtgt ttctgcatgt tctccattcc gctcctgatg tgcaggattg cccagaatgc     120
   acgctacagg aaaacccatt cttctcccag ccgggtgccc caatacttca gtgcatgggc     180
   tgctgcttct ctagagcata tcccactcca ctaaggtcca agaagacgat gttggtccaa     240
   aagaacgtca cctcagagtc cacttgctgt gtagctaaat catataacag ggtcacagta     300
   atggggggtt tcaaagtgga gaaccacacg gcgtgccact gcagtacttg ttattatcac    360
   aaatcttaa                                                             369
<210> 4
   <211> 474
   <212> DNA
   <213> Homo sapiens
<220>
   <221> misc_feature
   <223> wild-type nucleic acid sequence coding for the beta-chain of human FSH
<400> 4
   aggatccccg ggctacctcc ccgcggggag gcgcgcccct taattaagcc gccaccatga     60
   agacactcca gtttttcttc cttttctgtt gctggaaagc aatctgctgc aatagctgtg    120
   agctgaccaa catcaccatt gcaatagaga aagaagaatg tcgtttctgc ataagcatca    180
   acaccacttg gtgtgctggc tactgctaca ccagggatct ggtgtataag gacccagcca    240
   ggcccaaaat ccagaaaaca tgtaccttca aggaactggt atatgaaaca gtgagagtgc    300
   ccggctgtgc tcaccatgca gattccttgt atacataccc agtggccacc cagtgtcact    360
   gtggcaagtg tgacagcgac agcactgatt gtactgtgcg aggcctgggg cccagctact    420
   gctcctttgg tgaaatgaaa gaataaacat gccatggcat gcgagctcga attc          474
<210> 5
   <211> 471
   <212> DNA
   <213> Artificial
<220>
   <223> codon-optimized nucleic acid sequence coding for the beta-chain of human FSH
<400> 5
   aggatccccg ggtacctccc cgcggggagg cgcgcccctt aattaagccg ccaccatgaa     60
   gaccctgcag ttcttcttcc tgttctgctg ctggaaggcc atctgctgca acagctgcga    120
   gctgaccaac atcaccatcg ccatcgagaa ggaggagtgc aggttctgca tcagcatcaa    180
   caccacctgg tgcgccggat actgctacac cagggacctg gtgtacaagg accccgccag    240
   gcccaagatc cagaagacct gcaccttcaa ggagctggtg tacgagaccg tgagggtgcc    300
   cggctgcgcc caccacgccg acagcctgta cacctacccc gtggccaccc agtgccactg    360
   cggcaagtgc gacagcgaca gcaccgactg caccgtgagg ggcctgggcc ccagctactg    420
   cagcttcggc gagatgaagg agtaatgacc atggcatgcg agctcgaatt c             471
<210> 6
   <211> 372
   <212> DNA
   <213> Artificial
<220>
   <223> codon-optimized nucleic acid sequence coding for the alfa-chain of human FSH
<400> 6
   cttaattaag ccgccagcat ggactactac aggaagtacg ccgccatctt cctggtgacc     60
   ctgagcgtgt tcctgcacgt gctgcacagc gccccagacg tgcaggactg ccccgagtgc    120
   accctgcagg agaacccatt cttcagccag cccggagccc ccatcctgca gtgcatgggc    180
   tgctgcttca gcagggccta ccccaccccc ctgaggagca agaagaccat gctggtgcag    240
   aagaacgtga ccagcgagag cacctgctgc gtggccaaga gctacaacag ggtgaccgtg    300
   atgggcggct tcaaggtgga gaaccacacc gcctgccact gcagcacctg ctactaccac    360
   aagagctaat ga                                                        372

## Description

The present invention relates to a liquid pharmaceutical composition comprising a follicle stimulating hormone polypeptide, and benzalkonium chloride and benzyl alcohol as preservatives. The composition further comprises optionally one or more other pharmaceutically acceptable excipients. In one embodiment, the composition contains methionine as an antioxidant. The composition shows a good storage stability and is especially useful for the prophylaxis and treatment of disorders and medical indications where follicle stimulating hormone preparations are considered as useful remedies.

Follicle stimulating hormone (FSH) is produced by the gonadotropic cells of the anterior pituitary and released into the circulation. FSH acts together with the luteinising hormone (LH) in the control of oocyte maturation in females and of spermatogenesis in males. Both FSH and LH belong to a family of heterodimeric glycoproteins which consist of two non-covalently linked α- and β-chains which are encoded by separated genes. Both the α- and β-chains are glycosylated. The α-subunit consists of 92 amino acid residues while the β-subunit consists of 111 amino acid residues, each of which has two potential asparagine-linked glycosylation sites.

Human FSH is used to treat women with unovulation, for stimulation of multifollicular development (superovulation) and in preparation for an assisted conception such as IVF, ICSI, GIFT or CIFT. Furthermore, human FSH is used to stimulate the maturation of follicles in women with a low or absent FSH production and for the stimulation of spermatogenesis in men with congenital or acquired hypogonadotropic hypogonadism.

Until the 1980s, a primary source of human FSH was urine-derived FSH isolated from urine of child-bearing aged women. A further purified form of high-purity, urine-derived FSH was introduced in the 1990s, and finally a recombinant FSH was developed and widely used since the year of 1998. With the advent of recombinant DNA technology, it became possible to produce human FSH in cell cultures transfected with the nucleic acid sequences coding for the α- and the β-chain. DNA sequences coding for the α- and the β-chain and methods for producing recombinant FSH have been disclosed in e.g. WO 88/10270, WO 86/04589 and EP 0 735 139.

Currently, there are two commercial recombinant human FSH products on the market in Germany, GONAL-f^{®} and Puregon^{®}, both of which are produced by expression of the DNA sequences coding for the human wild-type α- and β-chains in Chinese Hamster Ovary (CHO) cells.

Generally, proteins have a very short half-life, and undergo denaturation such as aggregation of monomers, dissociation of dimers, and adsorption on the surfaces of vessels, upon exposure to various factors such as unfavourable temperatures for the expression of protein activity, water, air interface, high pressure, physical/mechanical stress, organic solvents and microbial contamination.

Consequently, the denatured proteins lose intrinsic physicochemical properties and physiologically active effects. Denaturation of proteins is irreversible and therefore proteins, once denatured, can hardly recover their native properties to the initial state.

In particular, proteins which have a heterodimeric structure consisting of two different subunits and are administered in a trace amount of less than several hundred micrograms each time, such as human FSH, suffer from problems associated with the relatively high loss of proteins including the loss of proteins due to dissociation of dimers in aqueous solutions and adsorption of proteins on the inner surface of vessels. The dissociated proteins lose their own physiological activity, and the dissociated monomers are readily susceptible to aggregation. Further, the proteins adsorbed on the inner surface of vessels are also readily vulnerable to aggregation via the denaturation process. When they are administered into the human body the thus-denatured proteins may serve as the cause of formation of antibodies against naturally occurring proteins in the body.

Some of protein pharmaceuticals have overcome stability problems via lyophilisation (freeze-drying). Although these freeze-dried preparations are stable enough to guarantee sufficient shelf-lives, they have the disadvantage that prior to administration reconstitution is necessary. The patient therefore necessarily has to reconstitute the dried glycoprotein in a solvent (such as, water for injection) before use, which is a disadvantage and an inconvenience to the patient. In addition, the solvent must be provided together with the freeze-dried preparations of the FSH. For a patient, who needs injections of a FSH at regular times, for instance a patient receiving a daily dose of a recombinant human FSH for ovulation induction, it would be of importance that the gonadotropin formulation is easy to handle, to dose and to inject. The reconstitution of a freeze-dried gonadotropin preparation demands prudence and carefulness and should be avoided, if possible. It would facilitate the use of gonadotropins, if these glycoproteins could be produced and distributed as a stable solution to the patient who could inject the medicament directly without reconstitution. In addition, a freeze-drying process is a costly and time consuming process step, and it would be an advantage if this step could be avoided when preparing a gonadotropin formulation.

A need exists therefore in a ready-for-use injection preparation, having a sufficient stability to guarantee a reasonable shelf-life.

As an alternative measure to cope with such limitations, the stability of the protein may be improved by adding a stabilizer to the protein in solution state. As examples of useful protein stabilizers, there are known surfactants, proteins such as albumins, polysaccharides, amino acids, polymers, salts and the like. However, the most suitable stabilizer should be selected and used taking into consideration unique physicochemical properties of individual proteins. Furthermore, combined use of different stabilizers may bring about adverse side effects as supposed to expected effects, due to competitive action and adverse reaction between individual stabilizers. Moreover, the successful stabilization of proteins present in the solution requires careful attention and many efforts, since the individual stabilizers have a specific concentration range favourable for stabilization of the corresponding proteins.

FSH has been formulated in both single-dose and multi-dose liquid formats, in vials, carpoules or ampoules. Single-dose formats must remain stable and potent in storage prior to use. Multi-dose formats must not only remain stable and potent in storage prior to use, but must also remain stable, potent and relatively free of bacteria over the multiple-dose use regimen administration period, after the seal of the ampoule has been compromised. For this reason, multi-dose formats often contain a bacteriostatic agent.

GONAL-f^{®}, a ready-to-use liquid preparation of human recombinant FSH, is available as a solution for injection administered from an injection pen. The solution contains *m-*cresol as a preservative (ROTE LISTE 2007, No. 50 004). Puregon^{®} is also available as a ready-to-use liquid preparation for injection which contains benzyl alcohol as a preservative (ROTE LISTE 2007, No. 50 010).

FSH liquid preparations containing a preservative have also been described in the patent literature. International patent application WO-A1-00/04913 describes FSH and FSH variant formulations comprising a preservative selected from phenol, *m-*cresol, *p-*cresol, *o-*cresol, chlorocresol, benzyl alcohol, alkylparaben, benzalkonium chloride, benzethonium chloride, sodium dehydroacetate and thiomerosal. While the use of these preservatives is mentioned in a general manner, there is no disclosure as to a specific combination or mixture of preservatives in a liquid formulation of recombinant human FSH.

Further, international patent application WO 2004/037607 is directed to an aqueous formulation of human FSH comprising glycine, methionine, non-ionic surfactant and a phosphate buffer as stabilizers. In addition, the formulation may optionally include a preservative, mentioning benzyl alcohol, cresol, paraben and mixtures thereof.

International patent application WO 2004/087213 A1 describes liquid pharmaceutical compositions of FSH comprising a surfactant selected from Pluronic^{®} F77, Pluronic^{®} F87, Pluronic^{®} F88 and Pluronic^{®} F68. The composition may further comprise a bacteriostatic agent selected from phenol, *m*-cresol, *p*-cresol, *o-*cresol, chlorocresol, benzyl alcohol, alkylparaben, benzalkonium chloride, benzethonium chloride, sodium dehydroacetate and thiomerosal. Mixtures or combinations of bacteriostatic agents are not mentioned.

Thus, while it is desired to provide gonadotropin formulations containing preservatives, so that the formulation is suitable for multiple administration, the production of preserved pharmaceutical preparations containing human protein has proven to be difficult. It has been observed that when preservatives are used, these give rise to stability problems if the pharmaceutical preparations are stored for longer periods. In this process, the human proteins are inactivated and agglomerates are formed which may be the cause of the observed intolerance to the injected solutions. Usual processes for the production of preserved pharmaceutical formulations for infusion or injection purposes cannot be used in the case of active human protein ingredients, since the active substances are inactivated under the sterilization conditions in autoclaves at 1 2 1 °C and their structure is destroyed. It is also known that the usual preservatives used in pharmacy react with the active human protein ingredients and these are thereby inactivated. The type of preservative used plays an important role for the tolerance. All preservatives have a greater or lesser allergy rate. It is therefore crucial that the preservatives in the human protein formulation are selected in that good preservation is achieved, but also the disadvantages of specific preservatives in combination with a certain human glycoprotein are minimized.

There is therefore still an urgent need for the development of a novel liquid formulation of human recombinant FSH preserved by the presence of preservatives and which is capable of stably maintaining the activity of the human recombinant FSH for an extended period of time.

According to the present invention, this and further problems are solved by means of the features of the main claim.

Advantageous embodiments are defined in the sub-claims.

It has surprisingly been found that formulating a recombinant human FSH in a liquid pharmaceutical composition comprising FSH or a variant thereof as active agent and both benzalkonium chloride and benzyl alcohol as preservatives provides a good stability of the composition so that it can be used for single-dose as well as for multiple-dose use. The composition according to the invention can be stored without cooling for a prolonged period of time, without significant loss of activity and without significant degradation. It is assumed that the combination of benzalkonium chloride and benzyl alcohol as preservatives is particularly advantageous in connection with the glycohormone FSH, since it provides better preservation and ensures that disadvantageous interactions with FHS are kept to a minimum.

Unless otherwise indicated, the following definitions are intended to illustrate and define the meaning and scope of the various terms used to describe the present invention.

The term "FSH" refers to a follicle-stimulating hormone polypeptide as a full-length mature protein which includes, but is not limited to, human FSH or "hFSH", whether produced recombinantly or isolated from human sources, such as the urine of postmenopausal women. The protein sequence of the human glycoprotein and the protein sequence of the human FSH β-subunit are known to the skilled person from the scientific and patent literature (see e.g. WO 2004/087213).

The amino acid sequence of the α-chain of human FSH is depicted in SEQ ID No. 1, and the amino acid sequence of the β-chain of human FSH is depicted in SEQ ID No. 2 as attached to this specification. These amino acid sequences correspond to the wild-type amino acid sequences of the α- and the β-chain of human FSH as deposited under accession number J 00152 in the EMBL database and under accession number NM_000510 in the NCBI database, respectively.

The wild-type nucleic acid sequences coding for human FSH are shown in SEQ ID No. 3 (= α-chain) and No. 4 (= β-chain).

The recombinant FSH may be encoded by the wild-type nucleic acid sequence as naturally found in human, or it may be encoded by an altered nucleic acid sequence whose expression results in an FSH having the wild-type amino acid sequence, i.e. the wild-type protein sequence as naturally found in human.

The nucleic acid sequence coding for human FSH can, for example, be altered in such a way that one or both of the nucleic acid sequences which code for the α- and the β-chain of human FSH have been adapted to the codon usage in Chinese Hamster Ovary (CHO) cells in order to increase the expression level and yield of recombinant FSH in these host cells.

An example of nucleic acid sequences which code for human FSH and which have been modified with regard to the codon usage in CHO cells is described in the international patent application WO 2009/000913. The modified nucleic acid sequence coding for the β-chain of human FSH is the coding region of the nucleic acid sequence depicted in SEQ ID No. 5 (in SEQ ID No. 5 the coding region starts at nucleotide 56 and extends up to nucleotide 442), and the modified nucleic acid sequence coding for the α-chain of human FSH is the coding region of the nucleic acid sequence given in SEQ ID No. 6 (in SEQ ID No. 6 the coding region starts at nucleotide 19 and extends up to nucleotide 366). A CHO cell line containing a recombinant nucleic acid molecule comprising a first modified nucleic acid sequence coding for the β-chain of human FSH and a second modified nucleic acid sequence coding for the α-chain of human FSH was deposited on 28 March 2007 at the DSMZ in Braunschweig under deposit number DSM ACC2833.

In a preferred embodiment, the FSH liquid formulation according to the present invention contains a recombinant human wild-type FSH which is obtained by recombinant gene expression from FSH nucleic acid sequences which are modified with regard to codon usage in CHO cells with respect to both the β-chain of human FSH and the α-chain of FSH. In another preferred embodiment, the recombinant FSH is obtained by expression from the FSH nucleic acid sequences disclosed in WO 2009/000913.

The expression "FSH variant" is meant to encompass those molecules differing in amino acid sequence, glycosylation pattern or in inter-subunit linkage from human FSH but exhibiting FSH activity. Examples include CTP-FSH, a long-acting modified recombinant FSH, consisting of the wild-type α-subunit and a hybrid β-subunit in which the carboxy terminal peptide of hCG has been fused to the C-terminal of the β-subunit of FSH, as described in LaPolt et al. (1992) Endocrinology, 131, 2514-2520; or Klein et al. (2003) Human Reprod., 18, 50-56. Also included is a single-chain CTP-FSH, a single-chain molecule described by Klein et al. (2002) Fertility & Sterility, 77, 1248-1255. Other examples of FSH variants include FSH molecules having additional glycosylation sites incorporated into the α- and/or β-subunit, as disclosed in WO 01/58493, and FSH molecules with inter-subunit S-S bonds, as disclosed in WO 98/58957. Other examples of FSH variants are disclosed in WO 2004/087213, which are characterized by carboxy terminal deletions of the β-subunit. Other examples of FSH variants include FSH molecules having an altered degree of glycosylation compared to wild-type FSH due to changes in the amino acid sequence of the protein by which additional glycosylation site(s) are introduced or naturally occurring glycosylation site(s) are deleted.

Further, the FSH or FSH variant according to the invention can be an FSH molecule which has been modified by chemical moieties. Such FSH conjugates can for example comprise poly alkylen glycol (such as PEG), hydroxyalkyl starch (such as HES) or other polymeric moieties.

FSH heterodimers or FSH variant heterodimers can be produced by any suitable method, such as recombinantly, by isolation or purification from natural sources, as the case may be, or by chemical synthesis, or any combination thereof.

The use of the term "recombinant" refers to preparations of FSH or FSH variants that are produced through the use of recombinant DNA technology (see for example WO 85/41958). The sequences for genomic and cDNA clones of FSH are known for the α- and β-subunits of several species. Various methods of producing recombinant FSH or FSH variants using recombinant technology are described in the prior art, see for example European patent application EP 0 711 894 and European patent application EP 0 487 512.

The FSH or FSH variant used in accordance with the present invention may be produced not only by recombinant means, including from mammalian cells, such as Chinese Hamster Ovary (CHO) cells, but also may be purified from other biological sources, such as from urinary sources. Acceptable methodologies are disclosed in the prior art.

The recombinant human FSH may be purified from the host cell culture supernatant by one or more purification steps. Suitable purification methods are known to the skilled person and include ion exchange chromatography, hydrophobic interaction chromatography, hydroxyapatite chromatography, affinity chromatography and gel filtration. Methods for purifying recombinant human FSH are disclosed e.g. in WO 00/63248, WO 2006/051070 and WO 2005/063811.

In a preferred embodiment of the invention, the FSH or FSH variant corresponds to human FSH or originates from human FSH, respectively. In a preferred embodiment of the invention, the FSH or FSH variant has been produced by recombinant technology.

Most preferably, the FSH is human FSH which has been produced recombinantly, particularly preferably produced in Chinese Hamster Ovary cells transfected with a vector or vectors comprising DNA coding for the human glycoprotein α-subunit and the β-subunit of FSH, either encoded by SEQ ID No. 3 and 4 (= wild-type nucleic acid sequences) or by SEQ ID No. 5 and 6 (= codon-optimized nucleic acid sequences). DNA encoding the α- and β-subunits may be present on the same or different vectors.

Recombinant FSH has several advantages over its urinary counterpart. Culture and isolation techniques using recombinant cells permit consistency between batches. In contrast, urinary FSH varies greatly from batch to batch in such characteristics as purity, glycosylation pattern, sialylation and oxidation of the subunits. Due to greater batch-to-batch consistency and purity of recombinant FSH, the hormone can be readily identified and quantified using techniques such as isoelectric focussing (IEF). The ease with which recombinant FSH can be identified and quantified permits the filling of vials by mass of hormone (fill-by-mass) rather than filling by bioassay.

The term "FSH activity" refers to the ability of an FSH formulation to elicit biological responses associated with FSH, such as ovarian weight gain in the Steelman-Pohley assay (Steelman et al. (1953) Endocrinology 53, 604-616), or follicular growth in a female patient. Follicular growth in a female patient can be evaluated by ultrasound, for example, in terms of the number of follicles having a mean diameter of about 16 mm on day 8 of stimulation. Biological activity is evaluated with respect to an accepted standard for FSH.

The term "aqueous diluent" refers to a liquid solvent that contains water. Aqueous solvent systems may consist solely of water, or may consist of water plus one or more miscible solvents, and may contain dissolved solutes such as sugars, buffers, salts or other excipients. The more commonly used non-aqueous solvents are the short-chain organic alcohols, such as methanol, ethanol, propanol, short-chain ketones, such as acetone, and polyalcohols, such as glycerol. In a preferred embodiment of the invention, the aqueous diluent is water, i.e. the liquid ready-for-use formulation according to the invention is an aqueous formulation.

A "tonicity modifying agent" or "isotonicity agent" is a compound that is physically tolerated and imparts suitable tonicity to a formulation to prevent the net flow of water across cell membranes that are in contact with the formulation. Suitable isotonicity agents include, but are not limited to, glycerol, amino acids or proteins (e.g., glycine or albumin), salts (e.g., sodium chloride), sugars (e.g., dextrose, sucrose, trehalose and lactose) and sugar alcohols (e.g., mannitol and sorbitol).

The term "bacteriostatic" or "bacteriostatic agent" or "preservative" refers to a composition or substance added to a formulation to act as an anti-bacterial agent. A preserved FSH or FSH variant containing formulation of the present invention preferably meets statutory or regulartory guidelines for preservative effectiveness to be a commercially viable multi-use product, preferably in humans.

The bacteriostatics used in the formulations according to the invention are benzalkonium chloride and benzyl alcohol in combination. While it is possible to include further preservatives, such as phenol, *m*-cresol, *p-*cresol, *o-*cresol, chlorocresol, alkylparaben (methyl, ethyl, propyl, butyl and the like), benzethonium chloride, sodium dehydroacetate or thimerosal, in the composition, i.e. in addition to benzalkonium chloride and benzyl alcohol, it is preferred that only benzalkonium chloride and benzyl alcohol are present as preservatives.

The term "buffer" or "physiologically acceptable buffer" refers to solutions of compounds that are known to be safe for pharmaceutical or veterinary use in formulations and that have the effect of maintaining or controlling the pH of the formulation in the pH range desired for the formulation. Acceptable buffers for controlling pH at a moderately acidic pH to a moderately basic pH include, but are not limited to, such compounds as phosphate, acetate, citrate, arginine, TRIS, and histidine. Preferable buffers are phosphate buffers.

The term "phosphate buffer" refers to solutions containing phosphoric acid or salts thereof, adjusted to a desired pH. Generally, phosphate buffers are prepared from phosphoric acid, or a salt of phosphoric acid, including but not limited to sodium and potassium salts. Several salts of phosphoric acid are known in the art such as sodium and potassium monobasic, dibasic and tribasic salts of the acid. Salts of phosphoric acid are also known to occur as hydrates of the occurring salt. Phosphate buffers may cover a range of pHs, such as from about pH 4 to about pH 10, and preferred ranges from about pH 5 to about pH 9, and a most preferred range of at or about 6.0 to at or about 8.0, most preferably at or about pH 7.0. In a preferred embodiment the buffer system consists solely of a phosphate buffer, i.e. no other buffering agent than phosphate is present in the formulation.

The term "vial" refers broadly to a reservoir suitable for retaining FSH in liquid form in a contained sterile state. Examples of a vial as used herein include ampoules, cartridges, blister packages, or other such reservoir suitable for delivery of the FSH to the patient via syringe, pump (including osmotic), catheter, transdermal patch, pulmonary or transmucosal spray. Vials suitable for packaging products for parenteral, pulmonary, transmucosal, or transdermal administration are well known and recognized in the art.

The term "stability" refers to the physical, chemical, and conformational stability of FSH in the formulations of the present invention (including maintenance of biological activity). Instability of a protein formulation may be caused by chemical degradation or aggregation of the protein molecules to form higher order polymers, by dissociation of the heterodimers into monomers, deglycosylation, modification of glycosylation, oxidation (particularly of the α-subunit) or any other structural modification that reduces at least one biological activity of an FSH polypeptide included in the present invention.

A "stable" solution or formulation or pharmaceutical composition is one wherein the degree of degradation, modification, aggregation, loss of biological activity and the like, of proteins therein is acceptably controlled, and does not increase unacceptably with time. Preferably, the formulation retains at least at or about 80% of the FSH activity and at least over a period of 6 months at a temperature of at or about 2-8°C. FSH activity can be measured by using the Steelman-Pohley ovarian weight gain bioassay.

The term "treating" refers to the administration, follow-up, management and/or care of a patient for whom FSH administration is desirable for the purpose of follicle or testicular stimulation or any other physiological response regulated by FSH. Treating can thus include, but is not limited to, the administration of FSH for the induction or improvement of sperm quality, stimulation of testosterone release in the male, or follicular development or for ovulation induction in the female.

The expression "multi-dose administration" or "multi-dose use" is intended to include the use of a single vial, ampoule, carpoule or cartridge of an FSH formulation for more than one injection, for example 2, 3, 4, 5, 6 or more injections. The injections are preferably made over a period of at least at or about 12 hours, 24 hours, 48 hours etc., preferably up to a period of at or about 12 days. The injections may be spaced in time, for example, by a period of 6, 12, 24, 48 or 72 hours.

The inventors have found that by formulating FSH or FSH variants with both benzalkonium chloride and benzyl alcohol as a preservative, they obtain a formulation which shows a high protein stability with simultaneous good preservation. Furthermore it is assumed that FSH formulation compositions according to this invention are showing an improved local tolerance (e.g. no irritation at the point of injection) compared to FSH formulations known in the arts.

Benzalkonium chloride is preferably present in the formulation at a concentration that is sufficient to maintain FSH stability over the desired storage period (for example 6 to 12 to 24 months), and also at a concentration that is sufficient to prevent bacterial growth.

Preferably, the concentration of benzalkonium chloride in the liquid formulation according to the invention is at or about 0.005 mg/ml to at or about 0.05 mg/ml, more preferably at or about 0.01 mg/ml to at or about 0.04 mg/ml, most preferably at or about 0.02 mg/ml (0.002 % (w/v)).

Benzyl alcohol is preferably present in the formulation at a concentration that is sufficient to maintain FSH stability over the desired storage period (for example 6 to 12 to 24 months) and also at a concentration that is sufficient to prevent bacterial growth.

Preferably, the concentration of benzyl alcohol in the liquid formulations according to the invention is at or about 0.5 mg/ml to at or about 20.0 mg/ml, more preferably at or about 1.0 mg/ml to at or about 15.0 mg/ml, more particularly preferably at or about 5.0 mg/ml to about 12.0 mg/ml, most preferably at or about 10.0 mg/ml (1.0 % (w/v)).

The follicle stimulating hormone (FSH) within the liquid formulations according to the invention is preferably present at a concentration at or about 150 IU/ml to at or about 2,000 IU/ml, more preferably at or about 300 IU/ml to at or about 1,500 IU/ml, more particularly preferably at or about 450 IU/ml to at or about 750 IU/ml, most preferably at or about 600 IU/ml.

The specific in vivo bioactivity of the recombinant FSH is usually in the range of about 8,000 IU FSH/mg of protein to about 16,000 IU FSH/mg of protein. For example, the recombinant human FSH in the commercially available product Puregon (from Organon) has a specific bioactivity of about 10,000 IE/mg protein, and for Gonal-f from Serono the bioactivity of the recombinant human FSH is about 13,600 IE/mg protein.

The FSH activity may be determined by known methods relating to FSH and other gonadotropins. Such methods include e.g. enzyme immunoactivity assay (EIA) or reporter gene assays. The bioactivity is usually determined by the bioassay described in the European Pharmacopeia, 5th Edition for urine derived FSH. In doing so the bioactivity is estimated by comparing the effect of FSH in enlarging the ovaries of immature rats treated with chorionic gonadotrophin with the same effect of a Standard Preparation.

The concentration of FSH or FSH variant in the liquid pharmaceutical composition according to the invention is usually in the range of 10 to 200 µg/ml. If the composition is intended for multi-use administration, e.g. by using an injection pen, a useful concentration of FSH or FSH variant will be in the range of 30 to 150 µg/ml, preferably in the range of 40 to 100 µg/ml.

In general, the FSH concentration will depend on the use (single dose or multi dose), on the way of adminstration, on the administration tool and on the bioactivity of the FSH or FSH variant.

Preferably, FSH or FSH variant is the only pharmaceutically active agent present in the pharmaceutical composition, even though it is possible to include other gonadotropins, such as LH.

The pharmaceutical composition according to the invention may further comprise a surfactant, preferably a non-ionic surfactant, in order to prevent adsorption of FSH or FSH variant on the surface of the vial, ampoule, carpoule, cartridge or syringe. Herein, the non-ionic surfactant lowers surface tension of a protein solution, thereby preventing adsorption or aggregation of the proteins on the hydrophobic surface. Preferred examples of the non-ionic surfactant that can be used in the present invention may include a polysorbate-based non-ionic surfactant and a poloxamer-based non-ionic surfactant. These non-ionic surfactants may be used alone or in any combination thereof. Particularly preferred is a polysorbate-based non-ionic surfactant. Specific examples of the polysorbate-based non-ionic surfactant include polysorbate 20, polysorbate 40, polysorbate 60 and polysorbate 80. More particularly preferred are polysorbate 20 and polysorbate 80, most preferred is polysorbate 20. The polysorbate 20 has a relatively low-critical micelle concentration. Therefore, the polysorbate 20 not only reduces or prevents surface adsorption of the proteins even at low concentrations, but also inhibits a chemical degradation of the proteins. Use of high-concentration non-ionic surfactant in the liquid composition according to the invention is not appropriate. This is because the use of the non-ionic surfactant at high concentration results in interference effects, thus making it difficult to precisely evaluate the stability of the proteins, when the concentration determination or stability evaluation of the proteins is carried out using an analysis method such as UV-spectroscopy or isoelectrical focussing. Therefore, the aqueous formulation of the present invention contains the non-ionic surfactant in a concentration of less than 1.0 mg/ml and more preferably 0.05 to 0.5 mg/ml.

In a preferred embodiment, polysorbate 20 is the only surfactant present in the formulation.

Preferably, FSH formulations of the present invention have a pH between at or about 6.0 and at or about 8.0, more preferably at or about 6.5 to at or about 7.5, including about pH 6.8, pH 7.0, pH 7.2 and pH 7.4. The preferred buffer is phosphate, with preferred counter-ions being sodium or potassium ions.

Buffer concentrations in total solution can be vary between 5 mM, 10 mM, 50 mM, 100 mM, 150 mM, 200 mM, 250 mM and 500 mM. Preferably, the buffer concentration is at or about 50 mM. Particularly preferred is a buffer 50 mM in phosphate ions with a pH of 7.0.

The pH of the formulation can be adjusted by adding a suitable acid or base in an appropriate amount. In one embodiment, the pH of the liquid pharmaceutical composition is adjusted using NaOH.

Preferably, the formulations of the invention contain an antioxidant, such as methionine, sodium bisulfite, salts of ethylenediamine tetraacetic acid (EDTA), butylated hydroxytoluene (BHT), and butylated hydroxyl anisole (BAH). Most preferred is methionine. The antioxidant prevents oxidation of FSH (particularly the α-subunit).

Methionine in the liquid pharmaceutical composition is preferably present at a concentration of at or about 0.1 to at or about 1.0 mg/ml, more preferably at or about 0.2 to 0.8 mg/ml, most preferably at or about 0.5 mg/ml.

In a preferred embodiment, methionine is the only antioxidant present in the formulation of the invention.

Preferably, the formulations of the invention contain a mono- or disaccharide or a sugar alcohol as a stabilizer and tonicity adjusting agent, such as sucrose, dextrose, lactose, sorbitol and/or glycerol. Most preferred is a sugar alcohol, particularly preferably mannitol.

The sugar or sugar alcohol is preferably present at a concentration of at or about 1.0 to 10 mg/ml, most preferably at a concentration of at or about 5.0 mg/ml. In one embodiment, mannitol is present in the composition according to the invention in an amount of 5.0 mg/ml.

In a preferred embodiment, mannitol is the only tonicity adjusting agent present in the formulation of the invention.

In a preferred embodiment, the formulation according to the invention does not contain any glycine.

In one embodiment, the liquid pharmaceutical composition of the invention contains FSH or a variant thereof as active agent, Polysorbate 20 and/or Polysorbate 80 as surfactant, mannitol as tonicity modifier, phosphate as buffer, methionine as stabilising agent and benzyl alcohol and benzalkonium chloride as preservatives, and water, and no further excipients.

As noted above, the invention provides liquid formulations for single use and multi-dose use, containing a combination of at least two bacteriostatic agents. The formulations of the invention are suited for pharmaceutical or veterinary use.

In one embodiment, the invention provides an article of manufacture for human pharmaceutical use, comprising packaging material and a container comprising a solution of FSH or FSH variant and benzyl alcohol and benzalkonium chloride, optionally with buffers and/or other excipients, in an aqueous diluent, wherein said packaging material comprises written material which indicates that such solution may be held over a period of 24 hours or greater after the first use. The container is preferably a syringe, vial, infusion bottle, ampoule or carpoule. Most preferably, the container is a carpoule within an injection pen.

Before the first use, that is before the seal of the container, vial, ampoule, carpoule or cartridge has been broken, the formulations of the invention may be kept for at least at or about 6 months, 12 months or 24 months. Under preferred storage conditions, before the first use, the pharmaceutical compositions of the invention are kept away from bright light (preferably in the dark), at temperatures of at or about 2-8°C.

The formulation of the invention can be administered using recognized devices. Examples comprising these single vial systems include pen-injector devices for delivery of a solution such as known as, or from, EasyJect^{®}, GONAL-F^{®} Pen, Humaject^{®}, Novopen^{®}, B-D^{®} Pen, AutoPen^{®}, Follistim^{®}-Pen, Puregon^{®}-Pen and OptiPen^{®}.

Stable preserved formulations may be provided to patients as clear solutions. The solution may be for single use or it may be re-used multiple times and may suffice for a single or multiple cycles of patient treatment and thus provides a more convenient treatment regimen than currently available.

The aqueous formulation according to the invention is a ready-to-use solution, there is no reconstitution of the FSH preparation at any point in time.

FSH or FSH variant in the stable preserved formulation described herein may be administered to a patient in accordance with the present invention via a variety of delivery methods, including s.c. or i.m. injection, transdermal, pulmonary, transmucosal, implant, osmotic pump, cartridge, micro pump, oral, or other means appreciated by the skilled artisan, as well-known in the art.

The following examples are provided merely to further illustrate the preparation of the formulations and compositions of the invention. The scope of the invention shall not be construed as merely consisting of the following examples.

The invention also relates to a pharmaceutical container containing the liquid pharmaceutical composition of the invention. Suitable pharmaceutical containers are known from the prior art. The container may, for example, be a syringe, vial, infusion bottle, ampoule or carpoule. In a preferred embodiment, when the container is a syringe, the syringe is equipped with a needle protection system. Such needle protection systems which are well known from the prior art help to reduce the risk of injuries. In another embodiment, the container is a carpoule within an injection pen. The invention also relates to a method of preparing a liquid pharmaceutical composition according to the invention, wherein FSH or FSH variant as the active agent is formulated in an aqueous preparation comprising benzalkonium chloride and benzyl alcohol as preservatives and further pharmaceutical excipients.

In another aspect the invention relates to the use of a liquid pharmaceutical composition according to the invention for multi-dose administration. The pharmaceutical composition of the invention can be advantageously used in the treatment of infertility and other disorders in connection with FSH. In a preferred embodiment the FSH formulation according to the invention is used for the treatment of a human. However, in general the pharmaceutical composition of the invention can also be administered to other mammals, such as sheep, cow, pig or horse.

The pharmaceutical liquid formulation according to the invention was found to exhibit a very good storage stability. Within the scope of the present invention, the term "storage stable" is understood to mean that the content of active FSH or FSH variant still amounts to 80% or more of the initial concentration after three months of storage of the formulation at 25°C. Preferably, after storage for three months at 25°C, the remaining content of FSH activity still amounts to at least 85%, more preferably at least 90%, and most preferably at least 95% of the original activity.

The biological activity of the FSH or FSH variant can be estimated by comparing, under given conditions, its effect in enlarging the ovaries of immature rats treated with chorionic gonadotrophin with the same effect of an International Standard preparation or of a reference preparation calibrated in International Units (European Pharmacopeia, 5th Edition).

The measurement of FSH activity *in vitro* is e.g. described by Albanese et. al. (1994) Mol. Cell Endocrinol. 101:211-219.

The purity of the FSH or FSH variant used in the formulation according to the invention should be at least 95%, preferably at least 97%, more preferably at least 99% and most preferably more than 99%. The degree of purity can be determined by means of HPLC analysis. Suitable materials and protocols for conducting such analysis can be obtained from commercial suppliers such as Vydac or TOSOH Bioscience.

The components for formulating the solutions according to the invention can be obtained from conventional sources, for example from companies such as Sigma or Merck.

The production of the formulation of the invention can be performed according to conventional methods. The components of the formulation can be dissolved in an aqueous buffer. Alternatively, the FSH or FSH variant can already be obtained in an aqueous buffer as the result of the purification process.

Finally, the finished liquid formulation is filled into a suitable pharmaceutical container, where it is stored until administration.

The following examples are intended to illustrate the invention without limiting its scope.

### EXAMPLES

### Example 1

### Preparation of a recombinant human FSH by recombinant technologies

Recombinant human FSH is produced in transfected CHO host cells by standard methods. These methods include the generation of a CHO cell clone which produces recombinant human FSH from one or more recombinant nucleic acid molecules which code for the α-chain and the β-chain of human FSH, cultivation of the host cells under suitable conditions and purification of the recombinant human FSH from the cell culture. Suitable processes are described in the prior art, as mentioned above.

In a preferred embodiment, the recombinant human FSH is produced as described in the international patent application WO 2009/000913.

### Example 2

### Liquid FSH formulation

A liquid formulation comprising recombinant human FSH was prepared by formulating the following components in an aqueous phosphate buffer solution.

| Ingredient | |
|---|---|
| Recombinant human FSH | 600 IU/ml |
| Polysorbat 20 | 0.2 mg/ml |
| Sodium phosphate | 50 mM |
| Mannitol | 5.0 mg/ml |
| L-methionine | 0.5 mg/ml |
| Benzyl alcohol | 10.0 mg/ml |
| Benzalkonium chloride | 0.02 mg/ml |
| Water for injection | |
| PH | 7.0 |

The bioactivity of the recombinant human FSH was determined as about 10,000 IU/mg.

The pH value of the composition was adjusted by adding NaOH. All ingredients are of quality according to the European Pharmacopoeia (Ph. Eur.). The formulation has a tonicity of 254 mOsmol/kg.

In addition, two prior art FSH formulations were prepared as comparative formulations:
A) Comparative formulation Gonal-f: 10 mM sodium phosphate, 60 mg/ml sucrose, 0.1 mg/ml methionine, 0.1 mg/ml poloxamer 188, 3 mg/ml *m*-cresol, pH 7.0.
B) Comparative formulation Puregon: 50 mM sodium citrate, 50 mg/ml sucrose, 0.5 mg/ml methionine, 0.2 mg/ml polysorbate 20, 10 mg/ml benzyl alcohol, pH 7.0.

### Example 3

### Stability tests of the formulations according to the present invention

The aqueous preparations as described in Example 2 were aliquoted in 1 ml/vial and stored at 2-8°C, at 25°C and at 37°C. After storage for 12 weeks at 25°C and 9 weeks at 37°C samples were tested for various test parameters. Sufficient preservation was tested according to the European Pharmacopoeia, 5th edition. % recovery of FSH, % decrease of dimeric FSH (dissociation of FSH into monomers) and purity of FSH were measured by SE-HPLC and SDS-PAGE.

The formulation of the invention exhibited more than 95% recovery of recombinant human FSH under storage at 2-8°C, 25°C and 37°C. In addition, the formulation exhibited less than 5% decrease of dimeric FSH under storage at 2-8°C, 25°C and 37°C. Further, the formulation exhibited more than 95% purity of FSH under storage at 2-8°C, 25°C and 37°C.

From these results, it can be seen that the FSH aqueous formulation comprising Polysorbate 20 as surfactant, mannitol as tonicity modifier, phosphate as buffer, methionine as stabilising agent and benzyl alcohol and benzalkonium chloride as preservatives according to the invention prevents protein loss and denaturation and dissociation of the protein into the constituent monomers, and stabilizes FSH for a prolonged period of time. In addition, due to its beneficial preservation performance the formulation is a useful preparation for multi-dose administration.

The efficacy of antimicrobial preservation of the FSH formulations according to this invention was determined with a challenge test according to the European Pharmacopoeia, 5th Edition. The test consists of challenging the aqueous formulation compositions with a prescribed inoculum of defined microorganisms, storing the inoculated formulation at a prescribed temperature, withdrawing samples from the formulation at specified intervals of time and counting the organisms in the samples so removed. The preservative properties of the preparation are adequate if, there is a significant fall or at least no increase in the number of microorganisms.

The FSH formulations according to this invention showed a significant decrease in the number of microorganisms and demonstrated therefore very good preservative properties.

Further, it was observed that the formulations of the present invention have a stability that is comparable to the stability of the prior art comparative formulations Gonal-f and Puregon.

Sequence listing:
- SEQ ID No.1:: amino acid sequence of the α-chain of human FSH
- SEQ ID No. 2:: amino acid sequence of the β-chain of human FSH
- SEQ ID No. 3:: wild-type nucleic acid sequence coding for the α-chain of human FSH
- SEQ ID No. 4:: wild-type nucleic acid sequence coding for the β-chain of human FSH
- SEQ ID No. 5:: codon optimized nucleic acid sequence coding for the β-chain of human FSH
- SEQ ID No. 6:: codon optimized nucleic acid sequence coding for the α-chain of human FSH

## Claims

1. A liquid pharmaceutical composition comprising follicle-stimulating hormone (FSH) or a variant thereof, and both benzalkonium chloride and benzyl alcohol as preservatives.

2. Liquid pharmaceutical composition according to claim 1, wherein benzalkonium chloride is present in a concentration of 0.005 - 0.03 mg/ml and benzyl alcohol is present in a concentration of 5.0 to 12.0 mg/ml.

3. Liquid pharmaceutical composition according to claim 1 or 2, wherein FSH or a variant thereof is present in a concentration of 10 to 200 µg/ml.

4. Liquid pharmaceutical composition according to any of the preceding claims, further comprising methionine as an antioxidant.

5. Liquid pharmaceutical composition according to any of the preceding claims, further comprising a surfactant.

6. Liquid pharmaceutical composition according to claim 5, wherein the surfactant is a polyoxyethylene sorbitan alkylester.

7. Liquid pharmaceutical composition according to any of the preceding claims, further comprising a tonicity modifying agent.

8. Liquid pharmaceutical composition according to claim 7, wherein the tonicity modifying agent is sugar alcohol or sugar.

9. Liquid pharmaceutical composition according to any of the preceding claims having a pH in the range of 6.5 to 7.5.

10. Liquid pharmaceutical composition according to any of the preceding claims, further comprising a physiologically acceptable buffering agent.

11. Liquid pharmaceutical composition according to any of the preceding claims, wherein the composition contains FSH or a variant thereof as active agent, Polysorbate 20 and/or Polysorbate 80 as surfactant, mannitol as tonicity modifier, phosphate as buffer, methionine as stabilising agent and benzyl alcohol and benzalkonium chloride as preservatives, and no further excipients.

12. A pharmaceutical container containing a liquid pharmaceutical composition according to any of the preceding claims.

13. Method for preparing a liquid pharmaceutical composition according to any of claims 1 to 11, wherein FSH or a variant thereof as the active agent is formulated in an aqueous preparation comprising both benzalkonium chloride and benzyl alcohol as preservatives and further pharmaceutical excipients.

14. Method for manufacturing a packaged pharmaceutical composition comprising placing a solution comprising FSH or a variant thereof, and benzalkonium chloride and benzyl alcohol in a vial, ampoule, carpoule or cartridge.

15. A liquid pharmaceutical composition according to any of claims 1 to 11 for use in multi-dose administration.

## Patentansprüche

1. Flüssige pharmazeutische Zusammensetzung umfassend Follikelstimulierendes Hormon (FSH) oder eine Variante davon, und sowohl Benzalkoniumchlorid als auch Benzylalkohol als Konservierungsmittel.

2. Flüssige pharmazeutische Zusammensetzung nach Anspruch 1, wobei Benzalkoniumchlorid in einer Konzentration von 0,005 - 0,03 mg/ml vorhanden ist und Benzylalkohol in einer Konzentration von 5,0 - 12,0 mg/ml vorhanden ist.

3. Flüssige pharmazeutische Zusammensetzung nach Anspruch 1 oder 2, wobei FSH oder eine Variante davon in einer Konzentration von 10 bis 200 µg/ml vorhanden ist.

4. Flüssige pharmazeutische Zusammensetzung nach einem der vorangehenden Ansprüche, weiterhin umfassend Methionin als Antioxidans.

5. Flüssige pharmazeutische Zusammensetzung nach einem der vorangehenden Ansprüche, weiterhin umfassend ein oberflächenaktives Mittel.

6. Flüssige pharmazeutische Zusammensetzung nach Anspruch 5, wobei das oberflächenaktive Mittel ein Polyoxyethylensorbitanalkylester ist.

7. Flüssige pharmazeutische Zusammensetzung nach einem der vorangehenden Ansprüche, weiterhin umfassend ein Tonizität-modifizierendes Mittel.

8. Flüssige pharmazeutische Zusammensetzung nach Anspruch 7, wobei das Tonizität-modifizierende Mittel ein Zuckeralkohol oder Zucker ist.

9. Flüssige pharmazeutische Zusammensetzung nach einem der vorangehenden Ansprüche mit einem pH im Bereich von 6,5 bis 7,5.

10. Flüssige pharmazeutische Zusammensetzung nach einem der vorangehenden Ansprüche, weiterhin umfassend eine physiologisch akzeptable Puffersubstanz.

11. Flüssige pharmazeutische Zusammensetzung nach einem der vorangehenden Ansprüche, wobei die Zusammensetzung FSH oder eine Variante davon als Wirkstoff, Polysorbat 20 und/oder Polysorbat 80 als oberflächenaktives Mittel, Mannitol als Tonizität-modifizierendes Mittel, Phosphat als Puffer, Methionin als Stabilisierungsmittel und Benzylalkohol und Benzalkoniumchlorid als Konservierungsmittel enthält, und keine weiteren Hilfsstoffe.

12. Pharmazeutisches Behältnis enthaltend eine flüssige pharmazeutische Zusammensetzung nach einem der vorangehenden Ansprüche.

13. Verfahren zur Herstellung einer flüssigen pharmazeutischen Zusammensetzung nach einem der Ansprüche 1 bis 11, wobei FSH oder eine Variante davon als Wirkstoff in einer wässrigen Zubereitung formuliert wird, die sowohl Benzalkoniumchlorid als auch Benzylalkohol als Konservierungsmittel und weitere pharmazeutische Hilfsstoffe umfasst.

14. Verfahren zur Herstellung einer verpackten pharmazeutischen Zusammensetzung, umfassend das Platzieren einer Lösung umfassend FSH oder eine Variante davon und Benzalkoniumchlorid und Benzylalkohol in einer Flasche (vial), Ampulle, Karpule oder Kartusche.

15. Flüssige pharmazeutische Zusammensetzung nach einem der Ansprüche 1 bis 11 zur Verwendung in Mehrfachdosis-Verabreichung.

## Revendications

1. Composition pharmaceutique liquide comprenant l'hormone de simulation folliculaire (FSH) ou une variante de celle-ci, et à la fois du chlorure de benzalkonium et de l'alcool benzylique comme agents conservateurs.

2. Composition pharmaceutique liquide selon la revendication 1, où le chlorure de benzalkonium est présent dans une concentration de 0,005 à 0,03 mg/ml et l'alcool benzylique est présent dans une concentration de 5,0 à 12,0 mg/ml.

3. Composition pharmaceutique liquide selon la revendication 1 ou 2, où la FSH ou une variante de celle-ci est présente dans une concentration de 10 à 200 µg/ml.

4. Composition pharmaceutique liquide selon l'une quelconque des revendications précédentes, comprenant en outre de la méthionine comme antioxydant.

5. Composition pharmaceutique liquide selon l'une quelconque des revendications précédentes, comprenant en outre un surfactant.

6. Composition pharmaceutique liquide selon la revendication 5, où le surfactant est un alkylester polyoxyéthylénique de sorbitanne.

7. Composition pharmaceutique liquide selon l'une quelconque des revendications précédentes, comprenant en outre un agent modificateur de tonicité.

8. Composition pharmaceutique liquide selon la revendication 7, où l'agent modificateur de tonicité est de l'alcool de sucre ou du sucre.

9. Composition pharmaceutique liquide selon l'une quelconque des revendications précédentes, ayant un pH compris entre 6,5 et 7,5.

10. Composition pharmaceutique liquide selon l'une quelconque des revendications précédentes, comprenant en outre un agent tampon physiologiquement acceptable.

11. Composition pharmaceutique liquide selon l'une quelconque des revendications précédentes, où la composition contient la FSH ou une variante de celle-ci comme agent actif, du polysorbate 20 et/ou du polysorbate 80 comme surfactant, du mannitol comme modificateur de tonicité, du phosphate comme tampon, de la méthionine comme agent stabilisateur et de l'alcool benzylique et du chlorure de benzalkonium comme agents conservateurs, et pas d'autres excipients.

12. Récipient pharmaceutique contenant une composition pharmaceutique liquide selon l'une quelconque des revendications précédentes.

13. Procédé d'élaboration d'une composition pharmaceutique liquide selon l'une quelconque des revendications 1 à 11, où la FSH ou une variante de celle-ci comme agent actif est formulée dans une préparation aqueuse comprenant à la fois du chlorure de benzalkonium et de l'alcool benzylique comme agents conservateurs et d'autres excipients pharmaceutiques.

14. Procédé de fabrication d'une composition pharmaceutique emballée, comportant le placement d'une solution comprenant la FSH ou une variante de celle-ci, et du chlorure de benzalkonium et de l'alcool benzylique, dans un flacon, une ampoule, une carpoule ou une cartouche.

15. Composition pharmaceutique liquide selon l'une quelconque des revendications 1 à 11, pour usage en administration multidose.
